(19) **Europäisches Patentamt**

**European Patent Office**

**Office européen des brevets**

(11) Publication number: **0 299 208 B1**

(12) # EUROPEAN PATENT SPECIFICATION

(45) Date of publication of patent specification: **01.09.93** (51) Int. Cl.⁵: **A61N 1/365**

(21) Application number: **88109322.3**

(22) Date of filing: **10.06.88**

(54) **Rate responsive pacemaker.**

(30) Priority: **17.06.87 SE 8702523**

(43) Date of publication of application:
**18.01.89 Bulletin 89/03**

(45) Publication of the grant of the patent:
**01.09.93 Bulletin 93/35**

(84) Designated Contracting States:
**DE FR GB IT NL SE**

(56) References cited:
**EP-A- 0 133 828      EP-A- 0 140 472**
**EP-A- 0 178 528      EP-A- 0 203 027**
**EP-A- 0 225 839      US-A- 4 726 383**
**US-A- 4 730 619**

(73) Proprietor: **Siemens Elema AB**
**Röntgenvägen 2**
**S-171 95 Solna 1(SE)**

(84) Designated Contracting States:
**SE**

(73) Proprietor: **SIEMENS AKTIENGESELLSCHAFT**
**Wittelsbacherplatz 2**
**D-80312 München(DE)**

(84) Designated Contracting States:
**DE FR GB IT NL**

(72) Inventor: **Moberg, Lennart**
**Kastrupg. 3,**
**S-16342 Spanga(SE)**
Inventor: **Hedin, Asa**
**Svearaegen 87**
**S-11350 Stockholm(SE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3. 10 / 3.6 / 3.3. 1)

## Description

This invention relates to a method for finding the parameters in a functional relationship between the stimulation frequency in an implantable physiological stimulating device, in particular a heart pacemaker, and a sensor signal indicative of the physical load condition of a patient.

Recently, pacemakers including a control system for matching the stimulation frequency of a heart pacemaker to the varying work load condition of a patient, generally referred to as rate responsive pacemakers, have been disclosed. Typically, rate responsive pacemakers rely on the sensing of a variable related to and indicative of the work load of a patient. Such variables are for instance blood oxygen saturation, blood PH, movement and force. The functional relationship between the sensed variable and the stimulation frequency could be linear or non-linear, depending on e.g. sensed variable, sensor type and sensor signal processing.

Irrespective of the type of functional relationship, linear or non-linear as the case may be, the parameters in the function must be set to values which are optimal to the individual pacemaker patient, as the sensor output signals varies from patient to patient depending on general fitness, body constitution, pacemaker placement, etc..

In the prior art these technical parameters were directly set, as illustrated in an example of a prior art pacemaker disclosed in US-A-4,428,378. That known pacemaker has a sensor of the (piezo electric) force type, and varies the stimulation frequency in response to sensed activity as a linear function with the slope as a programmable parameter. The slope parameter value is set by the physician and transmitted to the pacemaker by telemetry, to control pacemaker interaction with the patient.

However, directly setting the parameter values presents a problem to the physician as the technical character of these parameters, exemplified by slope above, render their values to lack immediate connection to the corresponding heart rate, i.e. the quantity familiar to the physician. Also, direct parameter value setting is inconvenient to the patient as an unexperienced physician must use a trial and error technique to find an optimal setting.

In consequence, the object of the present invention is to provide a method in which the parameter values are found in response to desired stimulation frequency settings, and thus provide for a more fast, safe and convient parameter setting.

Generally speaking, it is mathematically well known that any function between two variables can be determined when a sufficient number of variable values is known. Therefore, a function relating the sensor signal or measured variable, m to stimulation frequency, $f_{stim}$, could be determined with regard to its parameters from a number of observed values $f_{stim l}$ to $f_{stim\ n}$ and $m_l$ to $m_n$ where n at least equals the number of parameters to be set. Thus, in principle, though time-consuming for a complex function, by observing $f_{stim}$ and m for a sufficient number of different work loads, parameter calculation is possible.

However, the inventors have observed that the typical relationship between the sensor signal m and the stimulation frequency $f_{stim}$ is or could be arranged to be a function of the linear or exponential (logaritmic) type, viz. can be expressed as $f_{stim} = k_1 \cdot m + k_2$ or $f_{stim} = k_1 \cdot exp(k_2 \cdot m)$ ($f_{stim} = k_1 \cdot log(k_2 \times m)$ where $k_1$ and $k_2$ are parameters to be determined.

Therefore, in practice, normally only two parameters must be determined, and consequently the variable values for only two workloads of the patient have to be observed. A further reduction in number of work load exposition to a patient occurs when a dependency of the type $m_2 = m_1 + C_1$ - ($C_1$ = constant) between different sensor signals exists. In this case, a linear or exponential (logaritmic) function requires only one work load exposition. Thus, in normal clinical work only one or two work load expositions (desired heart rate settings) are necessary, and the described method presents an advantageous way of parameter setting.

It should be noted however, that although especially advantageous for the linear or exponential (logaritmic) relationship, the method is fully workable for more complex functions.

The invention is defined in claim 1 and preferable embodiments thereof are defined in the dependent claims.

In order that the invention may be more readily understood reference will now be made to the accompanying drawings.

FIG 1 is a functional block diagram of a rate-responsive pacemaker.

FIG 2 is a graph illustrating the linear relationship between sensor signal and stimulation frequency.

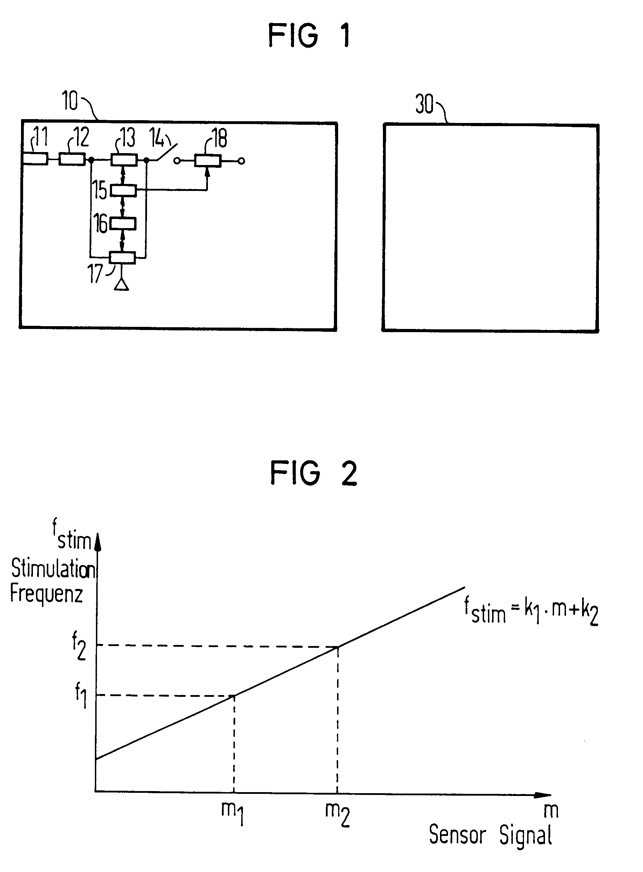

In FIG 1 the pacemaker is generally designated 10. A force sensor 11 is included in the pacemaker can. It should be noted here that also other sensor types and also other sensor placements could be used. The sensor signal is transformed in an A/D-converter block 12 into a signal m with a pulse duration proportional to the amplitude of the original sensor signal. Subsequently, the rate responsive parameters defining the constants of the function relating the sensor signal to the stimulation frequency, $f_{stim}$, are introduced in a parameter block 13. Block 13 is under the control of a logic and timing block 15, also controlling other functions of the pacemaker (e.g. output block 18), which, except for the rate responsive part, is a convential demand pacemaker.

Switch 14, enables a passive mode for the rate responsive function, viz. the pacemaker operates at the programmed minimum rate, while the rate which would have been present with switch 14 closed is still indicated from sensor 11.

The program memory 16 enables the programming signal and the calculated parameter values to be stored. That signal contains i.e. the desired stimulation frequency and is received from the programmer 30 through telemetry circuitry 17. The parameter calculating unit could either be contained in block 15 or programmer 30.

The setting of the rate responsive parameters is now described in connection with FIG 2. In FIG 2 a linear function ($f_{stim}$ = $k_1 \cdot m + k_2$ with $k_1$ and $k_2$ representingslope and threshold respectively) between sensor signal m and stimulation frequency is illustrated.

First, the patient is exposed to a low work load, for instance slow walking. This work load is correlated with a range of stimulation frequencies, e.g. 70 - 90 pulses per minute. The physician selects from this range one frequency $f_1$ appearing to be desirable to the individual pacemaker patient. The same level of work load corresponds to a sensor signal value $m_1$ indicative of said work load, which value is obtained from the pacemaker sensor.

Second a repetition of the above measures is carried out for another work load, for instance fast walking. This work load is correlated with another range of stimulation frequencies, e.g. 130 - 150 and the physician selects also here a desireable frequency, $f_2$. The corresponding sensor signal value is $m_2$.

The rate responsive parameters $k_1$ and $k_2$ can now be calculated either in the pacemaker circuitry or in the programmer by inserting $m_1$, $m_2$ and $f_1$, $f_2$ into the function. Thus, in accordance with the described method, slope and threshold are set in a way where the physician is only confronted with desired stimulation frequencies.

In case of a dependency between sensor signal for different work loads of the type described previously, viz. $m_2 = m_1 + C_1$ ($C_1$ = constant) only one stimulation frequency $f_1$ need to be selected, as the parameters can now be calculated from $f_1$, $m_1$ and the above dependency.

In a non-linear relationship of the exponential or logaritmic type, where the function also includes two parameters the same way of parameter setting applies, viz. for different work loads the values $m_1$, $m_2$ and $f_1$, $f_2$ are established and inserted into the function. Also here the dependency $m_2 = m_1 +$ constant may occur and is handled as in the linear case just described.

The invention has been described for a heart pacemaker just by way of example, and it should be noted that it applies to any implantable physiological stimulating device with a similar relationship between sensor signal and stimulation frequency.

## Claims

1. A method for finding the parameters in a functional relationship between the stimulation frequency of an implantable physiological stimulating device, in particular a heart pacemaker, and a sensor signal indicative of the physical load condition of a patient, **characterized by** the following steps:

   a) selecting a first stimulation frequency in substantial correspondence with a first load condition

   b) obtaining a first sensor signal corresponding to said first load condition

   c) repeating, steps a) - b) for further load conditions corresponding in number to the number of said parameters, and resulting in further stimulating frequencies and sensor signals respectively corresponding to said further load conditions

   d) calculating the parameters from said functional relationship and said selected frequencies and obtained sensor signals.

2. A method in accordance with claim 1, **characterized by** that the number of parameters is two.

3. A method in accordance with claim 1, **characterized by** that the functional relationship is linear.

4. A method in accordance with claim 1, **characterized by** that the functional relationship is exponential or logaritmic.

5. A method in accordance with any preceding claim **characterized by** that the interdependence between consecutive sensor signals $m_1$, $m_2$ is a constant.

6. A method according to any preceding claim **characterized by** the further step of storing said calculated parameter values in a look-up table.

## Patentansprüche

1. Verfahren zum Ermitteln der Parameter in einem funktionalen Zusammenhang zwischen der Stimulationsfrequenz einer implantierbaren physiologischen Stimulierungsvorrichtung, insbesondere einem Herzschrittmacher, und einem Sensorsignal, das die physische Belastung eines Patienten anzeigt, **gekennzeichnet durch** die folgenden Schritte:

a) Auswählen einer ersten Stimulationsfrequenz, die im wesentlichen einer ersten Belastung zuordenbar ist

b) Bestimmen eines ersten Sensorsignals ensprechend der ersten Belastung

c) Wiederholen der Schritte a)-b) für eine der Anzahl der Parameter entsprechende Anzahl weiterer Belastungen, die in weiteren Stimulierungsfrequenzen und Sensorsignalen resultieren, die jeweils den weiteren Belastungen zuordenbar sind

d) Berechnen der Parameter aus dem funktionalen Zusammenhang, den gewählten Frequenzen und bestimmten Sensorsignalen.

2. Verfahren nach Anspruch 1, **dadurch gekennzeichnet,** daß die Anzahl der Parameter zwei ist.

3. Verfahren nach Anspruch 1, **dadurch gekennzeichnet,** daß der funktionale Zusammenhang linear ist.

4. Verfahren nach Anspruch 1, **dadurch gekennzeichnet,** daß der funktionale Zusammenhang exponentiell oder logarithmisch ist.

5. Verfahren nach einem der vorhergehenden Ansprüche, **dadurch gekennzeichnet,** daß die gegenseitige Abhängigkeit zwischen konsekutiven Sensorsignalen $m_1$, $m_2$ eine Konstante ist.

6. Verfahren nach einem der vorhergehenden Ansprüche, **gekennzeichnet durch** den weiteren Schritt der Speicherung der berechneten Parameterwerte in einer Tabelle.

## Revendications

1. Un procédé pour trouver les paramètres intervenant dans une relation fonctionnelle entre la fréquence de stimulation d'un dispositif de stimulation physiologique implantable, en particulier un stimulateur cardiaque, et un signal de capteur représentatif de la condition de charge physique d'un patient, caractérisé par les étapes suivantes :

a) on sélectionne une première fréquence de stimulation correspondant pratiquement à une première condition de charge,

b) on obtient un premier signal de capteur correspondant à cette première condition de charge,

c) on répète les étapes a) - b) pour des conditions de charge supplémentaires, en un nombre correspondant au nombre des paramètres précités, cette répétition donnant des fréquences de stimulation et des signaux de capteur supplémentaires, correspondant respectivement aux conditions de charge supplémentaires,

d) on calcule les paramètres à partir de la relation fonctionnelle précitée et des fréquences sélectionnées et des signaux de capteur obtenus.

2. Un procédé selon la revendication 1, caractérisé en ce que le nombre de paramètres est de deux.

3. Un procédé selon la revendication 1, caractérisé en ce que la relation fonctionnelle est linéaire.

4. Un procédé selon la revendication 1, caractérisé en ce que la relation fonctionnelle est exponentielle ou logarithmique.

5. Un procédé selon l'une quelconque des revendications précédentes, caractérisé en ce que la relation entre des signaux de capteur consécutifs $m_1$, $m_2$ correspond à une constante.

6. Un procédé selon l'une quelconque des revendications précédentes, caractérisé par l'étape supplémentaire qui consiste à enregistrer dans une table les valeurs de paramètres calculées.

# FIG 1

# FIG 2

$$f_{stim} = k_1 \cdot m + k_2$$

5